# EUROPEAN PATENT SPECIFICATION

(11) **EP 1 018 335 B1**
(45) Date of publication and mention of the grant of the patent: **10.09.2003**
(21) Application number: 99670002.7
(22) Date of filing: 06.01.1999
(51) Int. Cl.: A61K 9/16

(54) **Process for formulating granular matrices by precipitation of polymers in an acidic medium**
Herstellungsverfahren für granuläre Matrizen durch Polymerausfällung in sauren Lösungen
Procédé de formulation de matrices granulaires par précipitation de polymères en milieu acide

(43) Date of publication of application: 12.07.2000
(73) Proprietor: TECNIMEDE-SOCIEDADE TECNICO-MEDICINAL, S.A., 2685-338 Prior Velho (PT)
(72) Inventor: De Sousa Goucha Jorge, Pedro Manuel, Dr., 2670-399 LOURES (PT); Mendes Cerdeira, Ana Maria, Dr., 2700-098 AMADORA (PT); Leitao Das Neves Almeida, Antonio José, Prof., 1990-175 LISBOA (PT)
(74) Representative: Ferreira, Maria Silvina

(56) References cited:
- EP-A- 0 130 162
- EP-A- 0 212 751
- EP-A- 0 213 908

## Description

The present invention refers to the preparation and use of a new system for obtaining spherical particulates of the matrix type (microspheres and beads) having a grain size from 1 µm to 3000 µm and simultaneously using gastro-resistant polymers and slow release polymers that may contain one or more active molecules or an active molecule and a cyclodextrin.

The dripping phenomenon has been used in research works in order to obtain a large number of pharmaceutical formulations. However the methods including the preparation of matrix type spherical particulates based on dripping solutions and suspensions show large omissions which prevent their use on an industrial scale.

In 1993, Bodmeier *et al*. (Bodmeier, R. and Wang, J. *Journal of Pharmaceutical Sciences,* 82, 191, 1993)) described a process of preparing spherical particulates through ionotropic gelation. This method provides slow release particulates through the ionotropic gelation of the anionic polysaccharide sodium alginate by positively charged calcium ions.
In 1995, Iannuccelli et al (Iannuccelli, V. and Bernabei, M. T., *Drug Development and Industrial Pharmacy*, 21, 2307, 1995) also refers to a process based on the ionotropic gelation of sodium carboxymethylcellulose with aluminum chloride.
However, these preparation methods of spherical particulates refer to a disposable syringe as the dripping instrument and therefore cannot be used on an industrial scale. Another process, also using the dripping of solutions, is described by Jedras et al. and refers to the preparation of pellets from melted materials (Jedras, Z., Janicki, S. *Pharmazie*, 43, 215, 1988). On the other hand, microencapsulation techniques, such as solvent evaporation, require organic solvents for solubilising polymers, for instance, dichloromethane, ethyl acetate or chloroform, the high toxicity and high costs of which are well known. The spray drying process, largely used on industrial scale, provides the obtention of microparticles; however, the low production efficiencies is a great inconvenience thereof.

Recently, the dripping method was also used for preparing gastro-resistant HP₅₀ beads by precipitating a HP₅₀ polymer solution or suspension containing the drug in citric acid at 0°C (Zamboni et al., *International Journal of Pharmaceutics*, 125, 151, 1995).

For certain extended therapies, e.g., the administration of nonsteroidal antiinflammatory agents for treating rheumatic arthritis, it is a matter of concern the protection of the gastro-intestinal system and the reduction of the number of the daily administrations, from consideration of patient's comfort. However, when trying to apply this method for preparing simultaneously gastro-resistant and slow release beads, two essential problems were arisen:
1. the application of the method using known polymers that delay the drug release resulted in formulations, the releasing profile of which was unstable during the shelving period, even when a plasticizer was included;
2. the utilization of the method as described is only possible on a laboratorial scale, a perfectly standardized apparatus being necessary to make possible its utilization on an industrial scale.

The main advantages of the invention process, in comparison with the above mentioned methods, are as follows:
1. production on an industrial scale through the use of a specifically designed apparatus;
2. production of simultaneously gastro-resistant and slow release beads and microspheres having steady release profiles;
3. no utilization of any solvent or toxic material;
4. it is a one step production method not needing the coating step.

For a better understanding and elucidation of the process according to this invention, the following drawings are enclosed:
- Fig. 1.: is a schematic view of the used apparatus for preparing the invention compositions;
- Fig. 2.: shows the releasing rate curve for Nimesulide (%) from the invention composition of Example 1 in 0.1N HCl and phosphate buffer pH 8.0, versus time (hrs);
- Fig. 3: shows the solubilization rate curve for Nimesulide (%) from the invention composition of Example 3 in the same liquid, vs. time (hrs);
- Fig. 4: shows the releasing rate curves for Numesulide (%) from the invention compositions of Examples 1, 6, 7, and 8 in phosphate buffer pH 8.0, vs. time (hrs);

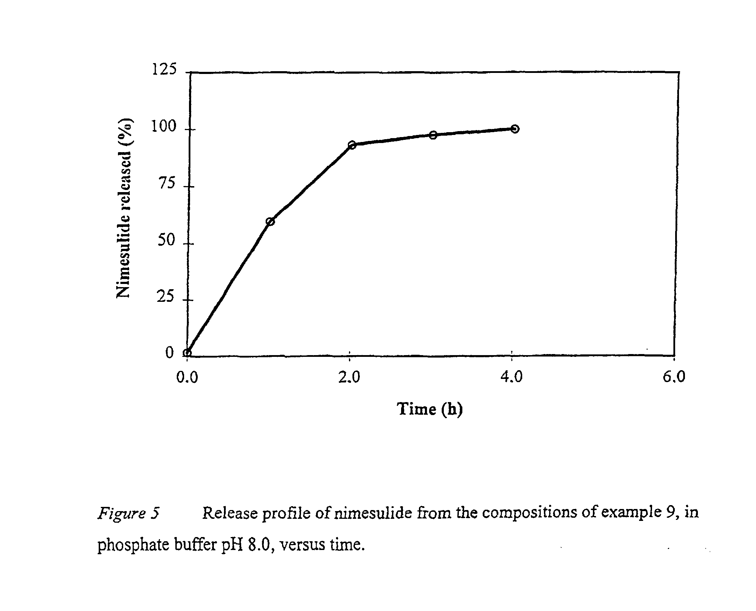

- Fig. 5: shows the releasing rate curve of Nimesulide (%) from the invention composition of Example 9 in the same liquid, vs. time (hrs);
- Fig. 6: represents a thermogram (DSC) of nabumetone-DM-β-cyclodextrin-HP₅₀ complex beads (1, and 3), and a thermogram of nabumetone (4);
- Fig. 7: represents the dissolution profiles of a formulation containing nabumetone-DM-β-cyclodextrin-HP₅₀ (◇) and of a formulation containing only nabumetone-HP₅₀(x);

First of all, the apparatus for preparing the formulations according to this invention will be briefly described.

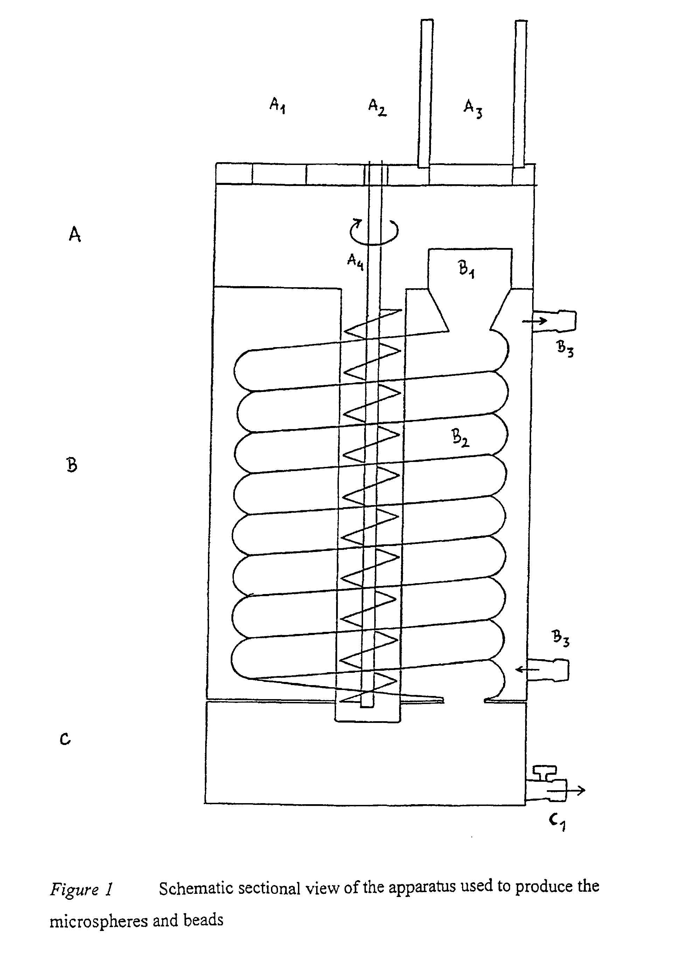

As it is shown by Fig. 1, said apparatus has two parts:
- **Lower part (C)**: a flat bottom vessel having a discharge cock (C₁), to remove the acidic solution from the inside of the apparatus.
- **Upper part (A+B)**: a vessel provided with three top holes:
   (A₁) for monitorization of variables such as temperature and pH;
   (A₂) for connection of the endless screw (A₄) to the motor;
   (A₃) which will be the bottom of a suport where some tubes or an nozzle are suspended.

Under this hole, a "funnel" (B₁) is provided. This "funnel" is connected to a coil (B₂) extending around the endless screw. This endless screw (A₄) will keep the liquid under rotation by the impelling action inwards, making the beads to move inside the coil in the direction of the bottom of the apparatus.

The part B has two cocks (B₃) for controlling the flow of a thermostatized liquid.

The upper part (A+B) and the lower part (C) are joined together so that overflows of liquid will be avoided during the production process.

This invention relates to a preparation method of simultaneously gastro-resistant and slow release microspheres and beads of a drug, or a combination of drugs or a cyclodextrin drug complex, characterized by the steps as follows:

According to the invention, a Process for the preparation of matrix type formulations based on the precipitation of alkaline solutions of gastro-resistant and slow release polymers in an acidic medium,
characterized in that it comprises:
1) preparation of an alkaline solution of polymers and plasticizer (if necessary) in which one or several drug molecules, or a drug and a cyclodextrin are dispersed or solubilized;
2) dripping said solution through a known small diameter tube or through a nozzle by compressed air action, into an acidic aqueous solution, the temperature range of which is 10-70°C;
3) drying said beads and microspheres using a suitable method (fluid bed drier, air drier, desiccator or lyophilization).

1) An alkaline solution essentially composed of a plasticizer (if necessary) and a mixture of gastro-resistant and slow release polymers is prepared. A drug, a mixture of drugs or a drug and a cyclodextrin mixture is added to this solution and, according to the solubility thereof, a solution or a suspension is obtained. The solid concentration (w/v) is limited by the viscosity of the solution or suspension.
2) A second liquid phase consisting of an aqueous acidic solution is prepared.
3) Next step of the preparation method includes the utilization of the apparatus schematised in Fig. 1.
4) The solution or suspension from step 1) is dipped through a known small diameter tube by means of a peristaltic pump.
5) Said solution or suspension 1) may also be added by a peristaltic pump through a nozzle with the aid of compressed air.
6) The dripping tube or nozzle under 4) and 5) is suspended from the support A₃ of the apparatus 3) which is filled-up with the solution prepared according to step 2). Under the hole of said tube or nozzle, a funnel (B₁) is provided where the drops fall; these drops are sucked down through a coil (B₂) connected to (B₁) and rotates around an endless screw (A₄). The endless screw (A₄) is connected to a motor (A₂) and promotes the acidic solution recycling under the impelling action inwards, pulling the formed particles. The part (B) has two cocks (B₃) that make possible the flow of a thermostatized liquid. The so formed particles fall into the lower part (C) where they are kept under agitation during the necessary period of time to have the needed strength for further processing.
   It is possible to scale-up the preparation apparatus dimensions to the industrial scale operating equipment.
7) The beads or microspheres are collected and washed with a deionized water volume enough to remove the acid adsorbed on the particles.
8) The washed particulates may be dried in a air drier, a fluid bed drier, in a desiccator or by freeze-drying in the case of microspheres.

As a characteristic feature of the used gastro-resistant polymers, they are soluble in an alkaline medium and precipitate in an acidic medium. Thus, it is possible the formation of matrices. Illustrative of useful polymers are hydroxypropylmethylcellulose phthalate, cellulose acetate phthalate and cellulose trimellitate acetate. The polymer minimal amount to be present in the solution or suspension to be dipped will be the necessary amount to obtain the gastro-resistance and the sphere formation; the maximal limit depends of the solution or suspension viscosity. Amounts within the range of 5 - 15% (w/v) are preferred.
Polymers giving slow release properties to matrices, in association to gastro-resistant polymers, cooperate and form the matrix; they may precipitate under acidic conditions and raise of temperature so that the spherical shape of particles is maintained. It is possible to use cellulose derivatives, such as hydroxypropylmethylcellulose or sodium carboxymethylcellulose, some methacrylic acid derivatives (Eudragit ® RS and RL) and as well as other known mixtures of the market, for instance, Surrelease ® (ethyl cellulose). The used amounts in the formulation recipe of the polymeric matrix are generally very low in dependence of the desired releasing profile and the used polymer.
Whenever the polymers need a maturation step, plasticizers should be added in order to decrease the duration and the temperature of maturation. These substances should not be much soluble in water so that they will not spread into the acidic solution. Illustrative examples of useful plasticizers are triethyl citrate, acetyl tributyl citrate and dibutyl sebacate. The plasticizers are to be used in a percentage ranging from 20% to 30% (w/w), based on the polymer amount needing maturation.
The concentration of the acidic solution must be high enough so that the precipitation of the above mentioned solution or suspension in steps 4) or 5) may take place. Percentages ranging from 5 to 20% (w/v) are recommended. The temperature range of the acidic solution is from 10°C to 70°C.
The drug molecules, or drug and cyclodextrin complexes for instance, nonsteroidal anti-inflammatory agents (e.g., Nimesulide, Etodolac, Piroxicam, Ibuprofen) have to be stable within the pH range of 1 - 8. In order to obtain perfect spherical matrix beads, drugs that are soluble in the polymeric dispersion 1) are used or, if they are insoluble, their crystal size is under 10 µm. For reducing the particle size, chemical methods (e.g., solubilization and precipitation of the drug molecules) or physical methods (e.g., ball mill, lyophilization) may be used.
The solution or the suspension to be dripped may be at the room temperature or heated up to 60° C in order to decrease its viscosity and make easier the dripping.
The diameter of the dripping tube is from 0.5 mm to 1 mm. The flow-rate of the peristaltic pump (4, 5) and the compressed air (5) pressure are dependent on the solution or suspension viscosity and the intended particulate diameter.
The rotation speed of the endless screw (A₄) depends on the intended retention period of time for the beads inside the coil (B₂) so that stiff enough particulates are obtained in the lower part of the apparatus.
The stirring speed within the lower part will be from 100 to 300 rpm.
The drying takes place between 25°C and 60°C. The present invention is illustrated by Examples 1 - 12.

### Example 1

Preparation of hydroxypropylmethylcellulose phthalate (HP₅₀) and carboxymethylcellulose sodium (NaCMC) beads containing Nimesulide

Preparation of the suspension to be dripped

| | |
|---|---|
| Deionized water | 100 ml |
| NaHCO₃ | 1.7 g |
| Plasticizer (triethyl citrate) | 0.114 g |
| NaCMC 0.380 | g |
| HP₅₀ | 12.0 g |
| Nimesulide | 6.6 g |
| NaOH (5M) | 6.0 ml |
| HCI (5M) | 4.5 ml |

The plasticizer is dispersed in 50 ml of alkaline sodium bicarbonate solution; then CaCMC and HP₅₀ are added. Separately, the Nimesulide is dispersed in 50 ml of deionized water using a high speed homogenizer; then the Nimesulide is solubilized in the NaOH solution and afterwards precipitated with HCI solution. After that, the polymeric solution is added to the drug solution. Simultaneously, an aqueous solution of 10% (w/v) citric acid is prepared. The end suspension is dripped through a 0.9 mm diameter tube into the citric acid solution at 60°C. The obtained particulates are maintained under mechanical stirring for 2 hours. Afterwards, they are washed with deionized water and dried in a fluidized bed stove at 40°C.

Table 1 shows the physico-chemical properties of the obtained product.

**Table 1**

| Formulation: Nimesulide 34.6%; HP₅₀ 62.8%; NaCMC 2.0% | | | | | |
|---|---|---|---|---|---|
| Mean value of diameter (± MSE) | Actual density (g cm⁻³) | Apparent density (g cm⁻³) | Porosity (%) | Hardness (N) | Encapsulation efficiency (%) |
| 1.29±0.11 | 1.3911±0.012 | 0.32038 | 76.9 | 5.033 ± 0.9266 | 88.8±1.5 |

The releasing tests *in vitro* according to USP 23 method for gastro-resistant compositions, confirm that the process based on dripping a suspension containing the association of both polymers and a drug molecule (e.g. Nimesulide) into an acidic solution results in a gastro-resistant slow release formulation.

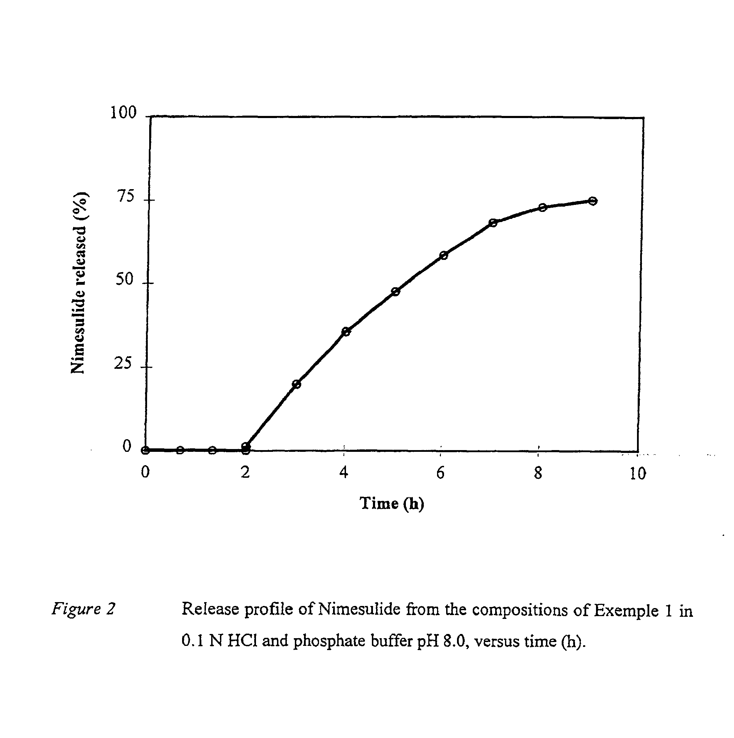

Fig. 2 shows the releasing rate curve for Nimesulide (%) from this composition in 0.1 M HCI and phosphate buffer pH 8.0, vs. time (hrs).

### Example 2: (version of Example 1)

The procedure is identical to Example 1, excepting that the drying temperature is 50°C.

### Example 3: (version of Example 1)

The procedure is identical to Example 1, excepting that the drying temperature is 60°C. Because the beads are dried at a higher temperature, the drying rate is much faster and therefore, a drug migration towards the surface of the polymeric matrix is caused and the release occurs much faster. On the other hand, all the physico-chemical properties of the product are quite different (Table 2).

**Table 2**

| Formulation: Nimesulide 34.6%; HP₅₀ 62.8%; NaCMC 2.0% | | | | | |
|---|---|---|---|---|---|
| Mean value of diameter (± MSE) | Actual density (g cm⁻³) | Apparent density (g cm⁻³) | Porosity (%) | Hardness (N) | Encapsulation efficiency (%) |
| 1.27±0.12 | 1.3005±0.0005 | 0.5636 | 56.67 | 8.65 ±0.13278 | 89.4 ± 3.3 |

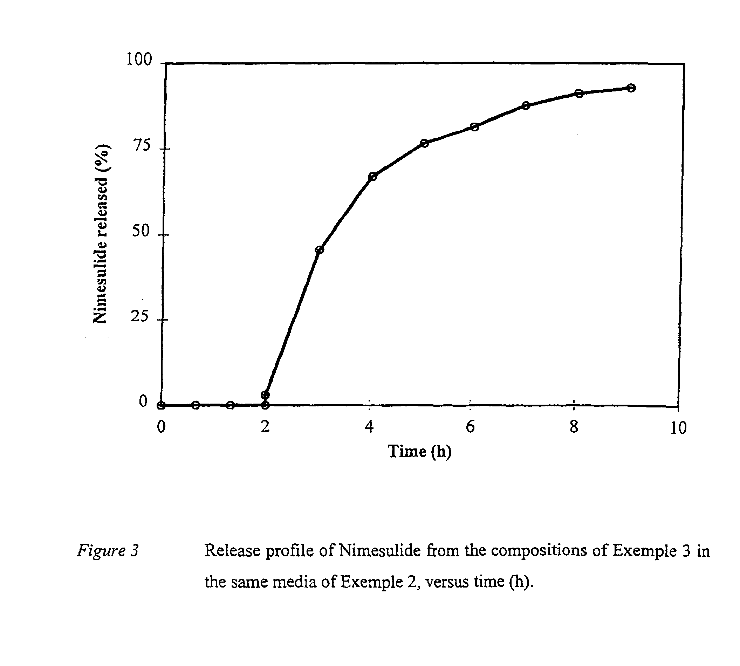

Fig. 3 shows the solubilization rate curve for Numesulide by same liquid, vs. time (hrs).

### Example 4: (version of Example 1)

The procedure is identical to Example 1, excepting that the drying took place in a desiccator.

### Example 5: (version of Example 1)

The procedure is identical to Example 1, but the drying took place in a air drier at the temperature of 40°C.

### Example 6: (version of Example 1)

The procedure is similar to Example 1, excepting that the used plasticizer is triacetine.

### Example 7: (version of Example 1)

The procedure is similar to Example 1, the used plasticizer being dibutyl sebacate.

### Example 8: (version of Example 1)

The procedure is similar to Example 1, but no plasticizer is added.

From Examples 1, 6, 7 and 8 it is possible to conclude that the uppermost parameter for obtaining constant release profiles is not the inclusion of a plasticizer in the polymeric matrix but the fact that the used temperature is 60°C.

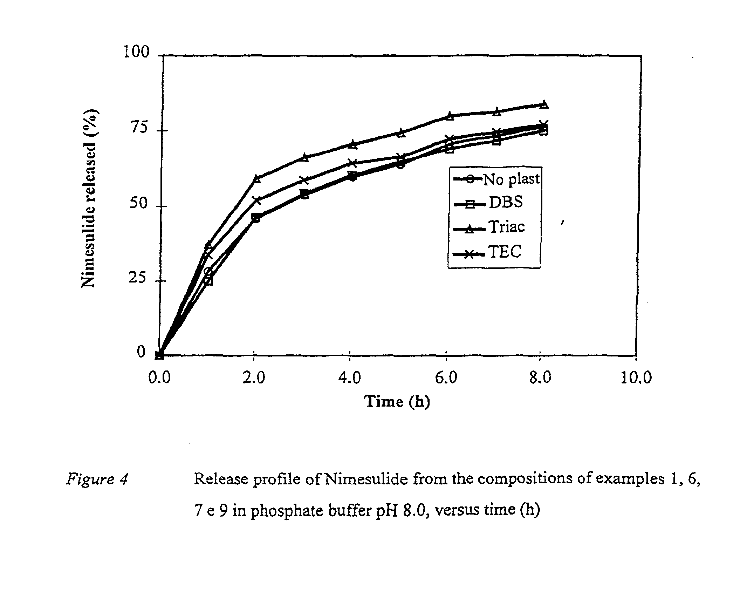

Fig. 4 shows the releasing rate curves for Nimesulide (%) from formulations of Examples 1, 6, 7 and 8 in phosphate buffer pH 8.0 solution.

### Example 9:

Preparation of HP₅₀ and NaCMC microspheres containg Nimesulide.

The procedure is similar to Example 1 but a nozzle and compressed air were used instead of the dripping tube.

### Example 10:

Preparation of HP₅₀ and NaCMC beads containing Etodolac

| | |
|---|---|
| Deionized water | 100 ml |
| NaHCO₃ | 1.7 g |
| Plasticizer (triethyl citrate) | 0.114 g |
| NaCMC 0.380 | g |
| HP₅₀ | 12.0 g |
| Etodolac | 6.6 g |

After preparing the polymeric solution containing plasticizer, Etodolac is dispersed in such a solution. Remaining procedure is similar to Example 1.

The obtained results are the same as the results for Nimesulide.

### Example 11:

Preparation of HP₅₀ and hydroxypropylmethyl cellulose (HPMC) beads containing Nimesulide

| | |
|---|---|
| Deionized water | 100 ml |
| NaHCO₃ | 1.7 g |
| Plasticizer (dibutyl sebacate) | 0.06 g |
| HPMC | 0.200 g |
| HP₅₀ | 12.0 g |
| Nimesulide | 6.6 g |
| NaOH (5M) | 5.0 ml |
| HCI (5M) | 4.5 ml |

The experimental procedure is identical to Example 1.

### Example 12:

Preparation of HP₅₀ beads containing a nabumetone/DM-β-cyclodextrin complex

| | |
|---|---|
| Deionized water | 100 ml |
| NaHCO₃ | 1.7 g |
| HP₅₀ | 15.0 g |
| Nabumetone | 2.5 g |
| DM-β-cyclodextrin | |

The HP₅₀ is solubilised in the sodium bicarbonate aqueous solution. To the latter, the drug and DM-β-cyclodextrin are added and stirred until a clear solution is obtained. This solution is then Dripped in the citric acid solution.

The granular matrices obtained comprises a nabumetone-DM-β-cyclodextrin complex as proved by the DSC of the beads.

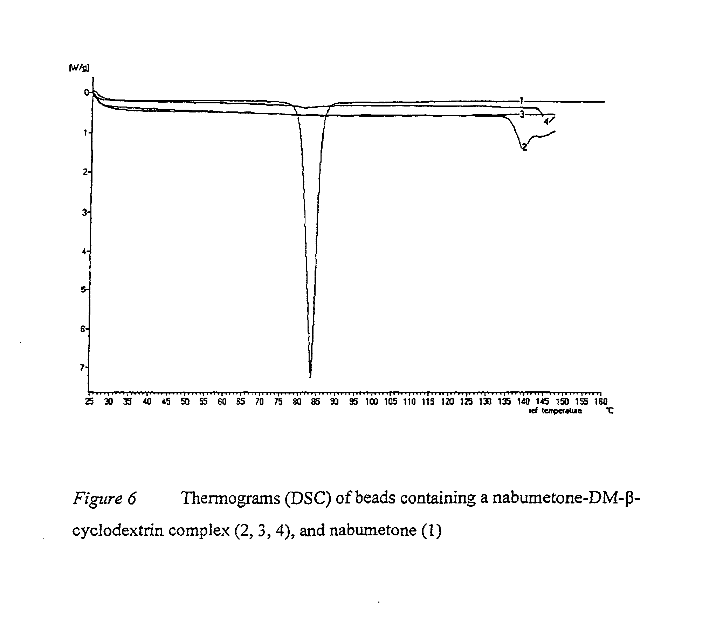

Fig. 6 shows the termograms (DSC) of nabumetone (1) and of beads containing nabumetone/DM-β-cyclodxtrin complex (2, 3 and 4). The absence of the nabumetone fusion peak in the beads is due to the nabumetone/ DM-β-cyclodextrin complex formation.

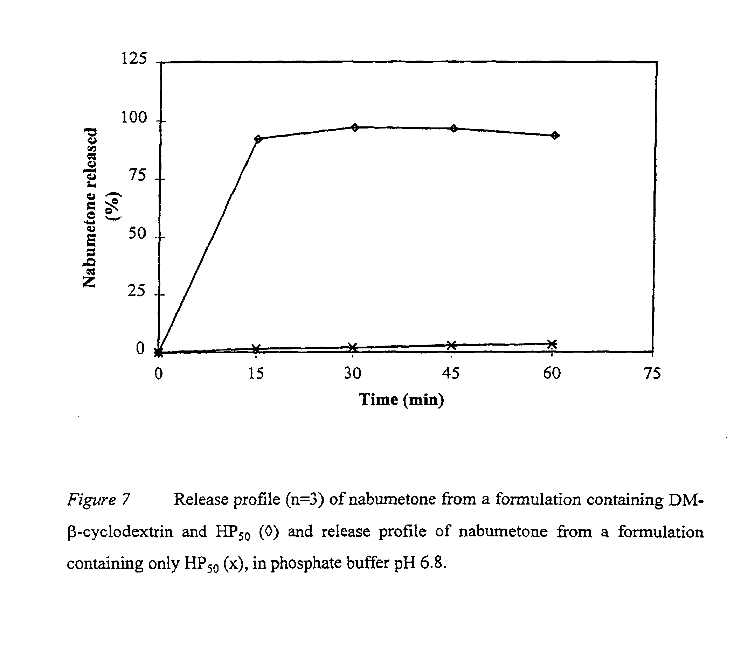

Fig. 7 shows the releasing rate profiles of a formulation containing nabumetone-HP₅₀ beads (x), and a formulation containing nabumetone-DM-β-cyclodextrin-HP₅₀ beads (◇), in phosphate buffer, pH 6.8.

## Claims

1. Process for the preparation of matrix type formulations based on the precipitation of alkaline solutions of gastro-resistant and slow release polymers in an acidic medium, **characterized in that** it comprises:
1) preparation of an alkaline solution of polymers and plasticizer (if necessary) in which one or several drug molecules, or a drug and a cyclodextrin are dispersed or solubilized;
2) dripping said solution through a known small diameter tube or through a nozzle by compressed air action, into an acidic aqueous solution, the temperature range of which is 10-70°C;
3) drying said beads and microspheres using a suitable method (fluid bed drier, air drier, desiccator or lyophilization).

2. Process according to claim 1, **characterized in that**, using the apparatus of Fig. 1, the following steps are carried out:
a solution or suspension of the drug, mixture of drugs or mixture of drug(s) and cyclodextrin is used by drops to the inside of the apparatus through a tube of known diameter held by the apparatus support (A₃), said drops falling in a funnel (B₁) filled up with an acidic solution; an endless screw (A₄) promotes the acidic solution recycling and impels the formed beads as a result of HP₅₀ precipitation across a coil (B₂); the so-formed beads and microspheres are kept under agitation until they reach the necessary strength for further processing; and lastly the beads are discharged washed with distilled water

3. Process according to claim 1, **characterized in that** beads containing any association of polymers selected form hydroxypropylmethylcellulose phtalate, hydroxypropylmethylcellulose acetate phtalate, cellulose trimellitate acetate, sodium carboxymethylcellulose, hydroxypropylmethylcellulose, ethylcellulose or any association of alkaline medium soluble and acidic medium precipitable polymers, and one or several drug molecules, are obtained.

4. Process according to claim 1, **characterized in that** microspheres containing the polymers and one or several drug molecules, or a drug and a cyclodextrin, as defined by claim 3, are obtained.

5. Process according to claim 1, **characterized in that**, as acidic aqueous solution, a solution of malic, tartaric, glycolic, malonic, maleic, fumaric, cinnamic, citric or mandelic acid is used.

6. Process according to claim 1, that allows to obtain a solid complex between a drug and a cyclodextrin.

7. Production of any pharmaceutical formulation (e. g. capsules or tablets) using the granular matrices as produced by the process according to any claim 1 to 5.

## Patentansprüche

1. Verfahren zur Herstellung von matrizenartigen Formulierungen basierend auf der Ausfällung von alkalischer Lösungen von gastroresistenten und verzögerten Freisetzungspolymeren in einem sauren Medium, **dadurch gekennzeichnet, daß** folgende Vorgänge durchgeführt werden:
1) die Zurbereitung einer alkalischen Lösung aus Polymeren und Weichmacher (wenn notwendig), in welcher eine oder mehrere Arzneimittelmoleküle oder ein Arzneimittel und ein Cyclodextrin dispergiert oder solubilisiert sind;
2) das Tropfen der genannten Lösung durch ein Rohr von bekanntem kleinen Durchmesser oder durch Drucklufttätigkeit durch eine Düse in eine saure wässerige Lösung, die eine Temperatur im Bereich von 10-70°C aufweist;
3) das Trocknen der genannten Körner und Mikrosphären anhand einer geeigneten Methode (Wirbelschichttrockner, Lufttrockner, Exsikkator oder Lyophilisierung).

2. Verfahren gemäß Anspruch 1, **dadurch gekennzeichnet, daß** die folgenden Schritte mit der in Abb. 1 aufgezeigten Vorrichtung durchgeführt werden:
eine Lösung oder eine Suspension des Arzneimittels, der Mischung der Arzneimitteln oder der Mischung von Arzneimittel(n) und Cyclodextrin wird durch ein Rohr von bekanntem Durchmesser, das durch die Halterung der Vorrichtung (A₃) befestigt ist, ins Innere der Vorrichtung getropft, wobei diese Tropfen in einen mit einer sauren Lösung gefüllten Trichter (B₁) fallen; eine Schnecke (A₄) fördert die Wiederverwertung der sauren Lösung und treibt die über die Ausfällung von HP₅₀ gebildeten Körner über eine Spule (B₂); die so gebildeten Körner und Mikrosphären werden durch Rührung in Bewegung gehalten bis sie die notwendige Festigkeit für die Weiterverarbeitung erreichen; und zuletzt werden die Körner entladen und mit destilliertem Wasser gewaschen.

3. Verfahren gemäß Anspruch 1, **dadurch gekennzeichnet, daß** Körner enthaltend eine beliebige Kombination von Polymeren, gewält aus Hydroxypropylmethylzellulose Phtalat, Hydroxypropylmethylzellulose Acetat Phtalat, Zellulose Trimellitat Acetat, Natriumcarboxymethylzellulose, Hydroxypropylmethylzellulose, Ethylzellulose oder irgendeine Kombination von in alkalischem Medium löslichen und in saurem Medium präzipitierbaren Polymeren und eine oder mehrere Arzneimitelmoleküle, erhalten werden.

4. Verfahren gemäß Anspruch 1, **dadurch gekennzeichnet, daß** Mikrosphären, die Polymere und eine oder einige Arzneimittelmoleküle, oder ein Arzneimittel und ein Cyclodextrin enthalten, wie in Anspruch 3 definiert, erhalten werden.

5. Verfahren gemäß Anspruch 1, **dadurch gekennzeichnet, daß** als saure wässerige Lösung, eine Apfel-, Wein-, Glycol-, Malon-, Malein-, Fumar-, Zimt-, Zitronenoder Mandelsäurelösung verwendet wird.

6. Verfahren gemäß Anspruch 1, **dadurch gekennzeichnet, daß** es die Gewinnung eines festes Komplexes zwischen einem Arzneimittel und einem Cyclodextrin ermöglicht .

7. Herstellung jeglicher pharmazeutischen Formulierung (z.B. Kapseln oder Tabletten) unter Verwendung der granularen Matrizen, die durch das Verfahren gemäß einem der Ansprüche 1 bis 5 erhalten werden.

## Revendications

1. Procédé pour la préparation des formulations de type matricielle basées en la précipitation des solutions alcalines des polymères gastrorésistantes et de libération prolongée en milieu acide, **caractérisé en ce qu'**il comprendre:
1) la préparation d'une solution alcaline des polymères et de plastifiant (au besoin) dans laquelle une ou plusieurs molécules de drogue, ou une drogue et une cyclodextrine sont dispersées ou solubilisées;
2) l'égouttement de ladite solution par un tube de diamètre petit connu ou par un bec par action de l' air comprimé, dans une solution aqueuse acide, dont l' intervalle de température est 10-70°C;
3) la séchage de lesdites granules et microsphères utilisant une méthode appropriée (séchoir à lit fluide, séchoir à air, dessiccateur ou lyophilisation).

2. Procédé selon la revendication 1, **caractérisé en ce que**, à l'aide de l'appareil de Fig. 1, les étapes suivantes sont réalisées: une solution ou une suspension de la drogue, du mélange des drogues ou du mélange d'une ou plusieurs drogues et de la cyclodextrine est conduit goutte à goutte à l'intérieur de l'appareil par un tube de diamètre connu tenu par l'appui d'appareil (A₃), tombant lesdites gouttes dans un entonnoir (B₁) rempli de solution acide; une vis sans fin (A₄) favorise la réutilisation de la solution acide et pousse les granules formés par la précipitation de HP₅₀ à travers un enroulement (B₂); les granules et les microsphères ainsi formés sont tenus sous l'agitation jusqu'à ce qu'ils atteignent la résistance nécessaire pour une transformation plus ultérieure; et finalement les granules sont déchargés et lavés avec l'eau destillée.

3. Procédé selon la revendication 1, **caractérisé en ce que** s' obtient des granules contenant quelconque association des polymères choisis parmi le phtalate de hydroxypropylmethylcellulose, le phtalate d'acétate de hydroxypropylmethylcellulose, l'acétate de trimellitate de cellulose, la carboxyméthylcellulose sodique, le hydroxypropylmethylcellulose, l'ethylcellulose ou l'une quelconque association des polymères solubles en milieu alcalin et précipitables en milieu acide, et une ou plusieurs molécules de drogue.

4. Procédé selon la revendication 1, **caractérisé en ce que** s'obtient des microsphères contenant les polymères et une ou plusieurs molécules de drogue, ou une drogue et une cyclodextrine, comme définie par la revendication 3.

5. Procédé selon la revendication 1, **caractérisé en ce que**, comme solution aqueuse acide, il est employée une solution d'acide malique, tartrique, glycolique, malonique, maléique, fumarique, cinnamique, citrique ou mandélique.

6. Procédé selon la revendication 1, **caractérisé en ce qu'**il permet obtenir un complexe solide entre une drogue et une cyclodextrine.

7. Production de quelconque formulation pharmaceutique (par exemple capsules ou comprimés) employant les matrices granulaires comme produites par le procédé selon l'une quelconque revendication 1 à 5.
